# EUROPÄISCHE PATENTSCHRIFT

(11) **EP 2 904 386 B1**
(45) Veröffentlichungstag und Bekanntmachung des Hinweises auf die Patenterteilung: **13.06.2018**
(21) Anmeldenummer: 13773605.4
(22) Anmeldetag: 28.09.2013
(51) Int. Cl.: G01N 33/00

(54) **VERFAHREN ZUR VERARBEITUNG VON MESSWERTEN EINES STICKOXID-SENSORS**
METHOD FOR PROCESSING MEASURED VALUES FROM A NITROGEN OXIDE SENSOR
PROCÉDÉ DE TRAITEMENT DE VALEURS DE MESURE D'UN DÉTECTEUR D'OXYDE D'AZOTE

(30) Priorität: 06.10.2012 DE 102012019633
(43) Veröffentlichungstag der Anmeldung: 12.08.2015
(73) Patentinhaber: Daimler AG, 70327 Stuttgart (DE)
(72) Erfinder: LAHR, Jochen, 70565 Stuttgart (DE)
(74) Vertreter: JENSEN & SON
(86) Internationale Anmeldenummer: PCT/EP2013/002925
(87) Internationale Veröffentlichungsnummer: WO 2014/053229

(56) Entgegenhaltungen:
- DE-A1- 10 049 685
- DE-A1- 10 309 422
- DE-A1-102008 005 640
- US-A1- 2009 158 813
- US-A1- 2010 257 846
- US-A1- 2011 252 767

## Beschreibung

Die Erfindung betrifft ein Verfahren zur Verarbeitung von Messwerten eines Stickoxid-Sensors nach dem Oberbegriff des Anspruchs 1.

Aus der DE 10 2008 005 640 A1 ist ein Verfahren zur Ermittlung einer Stickstoffdioxidkonzentration in einem Abgasstrang einer Brennkraftmaschine bekannt, bei welchem die Ausgangssignale zweier Stickoxidsensoren miteinander verglichen werden. Dabei ist der erste Stickoxidsensor vor und der zweite Stickoxidsensor hinter einem Abgasbehandlungselement mit der Fähigkeit Stickstoffmonoxid (NO) zu Stickstoffdioxid (NO₂) umsetzen zu können, angeordnet. Auf diese Weise kann eine bei üblichen Stickoxid-Sensoren vorhandene unterschiedliche Ansprechempfindlichkeit gegenüber NO und NO₂ ausgeglichen werden. Aus der US2011252767 A1 ist bekannt aus dem Vergleich zweier Messwerte von Stickoxidsensoren auf die korrekte Funktionsweise zu schließen. Es wird jedoch keine Korrekturmaßnahme offenbart. Aus der DE10309422 A1 ist ein Verfahren zur Kalibrierung von Stickoxidsensoren bekannt, wobei jedoch kein Vergleich zwischen mehreren Sensoren stattfindet. Aufgabe der Erfindung ist, ein Verfahren anzugeben, welches ein genaue Ermittlung eines Stickoxidgehalts im Abgas einer Brennkraftmaschine ermöglicht.

Diese Aufgabe wird durch ein Verfahren mit den Merkmalen des Anspruchs 1 gelöst.

Bei dem erfindungsgemäßen Verfahren werden Messwerte eines ersten und eines zweiten Stickoxid-Sensors verarbeitet. Der erste Stickoxid-Sensor ist stromauf eines Stickoxidreduktionskatalysators im Abgasstrang eines Kraftfahrzeugs angeordnet und der zweite Stickoxid-Sensor ist stromab des Stickoxidreduktionskatalysators angeordnet. Bei dem Verfahren werden wenigstens annähernd zeitgleich Messwerte des ersten und des zweiten Stickoxid-Sensors erfasst und miteinander verglichen, wobei eine Empfindlichkeit des ersten Stickoxid-Sensors und/oder des zweiten Stickoxid-Sensors in Abhängigkeit vom Ergebnis des Vergleichs verändert wird. Auf diese Weise ist es ermöglicht, unerwünschte Empfindlichkeitstoleranzen der Stickoxid-Sensoren, typischerweise verursacht durch Exemplarstreuungen, auszugleichen. Bei den Stickoxid-Sensoren handelt es sich bevorzugt um solche gleicher Bauart.

Die Empfindlichkeitsveränderung des ersten und/oder des zweiten Stickoxid-Sensors kann beispielsweise durch Veränderung der Verstärkung erfolgen. Die Änderung kann dabei direkt am Ausgangssignal des Sensors erfolgen, sie kann aber auch durch Signalkorrektur an einem Steuergerät erfolgen, an welchem der Sensor bzw. die Sensoren angeschlossen sind und welches die Signale des Sensors bzw. der Sensoren verarbeitet. Bei der Erfindung werden die Empfindlichkeit des ersten Stickoxid-Sensors und/oder des zweiten Stickoxid-Sensors derart verändert, dass der erste Stickoxid-Sensor und der zweite Stickoxid-Sensor wenigstens annähernd gleiche Empfindlichkeiten gegenüber NO und/oder NO₂ aufweisen. Typischerweise liefern die Sensoren ein Ausgangssignal, welches mit der Summe von NO und NO₂ d.h. mit der Konzentration von Stickoxiden (NOx) im Abgas korreliert. Bei dem Ausgangssignal handelt es sich somit um ein NOx-Konzentrations- bzw. Gehaltssignal. Der Vergleich der Messwerte des ersten und des zweiten Stickoxid-Sensors erfolgt unter Bedingungen, bei den am Ort der Sensoren gleiche Konzentrationen bzw. Gehalte von NOx im Abgas erwartet werden können. Bei der Empfindlichkeitsveränderung in Abhängigkeit des Signalvergleichs kann beispielsweise dann das Signal des Sensors, welcher eine niedrigere NOx-Konzentration anzeigt, auf das Signal des Sensors mit der höheren Anzeige angehoben werden. Umgekehrt kann vorgesehen sein, das Signal des NOx-Sensors mit dem höheren Messwert entsprechend abzusenken. Es kann jedoch auch vorgesehen sein, die Signale beider Sensoren auf einen mittleren Wert einzustellen. In den genannten Fällen weisen die Stickoxid-Sensoren nach Durchführung des Signalvergleichs und der Empfindlichkeitsveränderung wenigstens annähernd gleich Signale auf oder die Signale sind auf einen wenigsten annähernd gleichen Wert korrigiert.

Zur Vermeidung des Einflusses einer gegebenenfalls vorhandenen Querempfindlichkeit der Stickoxid-Sensoren ist es in der Erfindung vorgesehen, dass der Vergleich zu einem Zeitpunkt vorgenommen wird, bei dem am Einbauort des ersten Stickoxid-Sensors und am Einbauort des zweiten Stickoxid-Sensors wenigstens annähernd gleiche Konzentrationen von Kohlenwasserstoffen (HC) und/oder NOx im Abgas vorhanden sind. Erfindungsgemäß ist es vorgesehen, dass der Vergleich zu einem Zeitpunkt vorgenommen wird, bei dem am Einbauort des ersten Stickoxid-Sensors und am Einbauort des zweiten Stickoxid-Sensors die Konzentrationen von Kohlenwasserstoffen und/oder Ammoniak (NH₃) im Abgas vorgegebene Grenzwerte nicht überschreiten. Dies ermöglicht eine besonders wirksame Ausschaltung von Querempfindlichkeitseffekten der Sensoren in Bezug auf HC und NH₃. Insbesondere ist es vorgesehen, die Grenzwerte niedrig anzusetzen. Vorzugsweise liegen die Grenzwerte unterhalb von 20 ppm, besonders bevorzugt unterhalb von 10 ppm oder sogar unterhalb von 5 ppm.

In weiterer Ausgestaltung der Erfindung wird der Vergleich zu einem Zeitpunkt vorgenommen, bei dem die Abgastemperatur am Einbauort des ersten Stickoxid-Sensors und am Einbauort des zweiten Stickoxid-Sensors einen vorgebbaren Temperatur-Grenzwert überschreitet. Die Temperatur wird bevorzugt so gewählt, dass das thermodynamische NO - NO2-Gleichgewicht überwiegend auf der Seite von NO liegt. Dies ist bei Temperaturen oberhalb von etwa 350 °C der Fall. Der Vergleich wird daher bevorzugt bei einem Temperatur-Grenzwert von 400 °C, insbesondere von 450 °C und besonders bevorzugt bei einem Temperatur-Grenzwert von 500 °C für die Abgastemperatur vorgenommen. Ist ein Partikelfilter in der entsprechenden Abgasanlage verbaut, so sind diese Bedingungen meist erfüllt, wenn eine thermische Regeneration des Partikelfilters durch Rußabbrand mit Sauerstoff erfolgt. Der Vergleich erfolgt daher in einer weiteren vorteilhaften Ausgestaltung der Erfindung unmittelbar im Anschluss an eine solche thermische Partikelfilter-Regeneration.

Vorteilhafte Ausführungsformen der Erfindung sind in der Zeichnungen veranschaulicht und werden nachfolgend beschrieben. Dabei sind die vorstehend genannten und nachfolgend noch zu erläuternden Merkmale nicht nur in der jeweils angegebenen Merkmalskombination, sondern auch in anderen Kombinationen oder in Alleinstellung verwendbar, ohne den Rahmen der vorliegenden Erfindung zu verlassen.

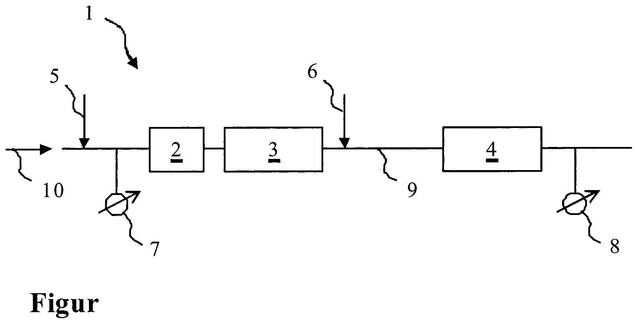

Dabei zeigt die einzige Figur ein schematisches Blockbild einer an eine Kraftfahrzeug-Brennkraftmaschine angeschlossenen Abgasreinigungsanlage.

In der Figur ist lediglich grob schematisch eine Abgasreinigungsanlage 1 einer Kraftfahrzeug-Brennkraftmaschine, insbesondere eines Dieselmotors dargestellt. Abgas der nicht dargestellten Brennkraftmaschine tritt entsprechend der mit dem Pfeil 10 gekennzeichneten Richtung in die Abgasreinigungsanlage 1 ein. Diese weist in Abgasströmungsrichtung gesehen hintereinander einen Oxidationskatalysator 2, einen Partikelfilter 3 und einen SCR-Katalysator 4 in einer Abgasleitung 9 auf. Der SCR-Katalysator 4 kann auch als zweiflutige Katalysatoranordnung von zwei parallel geschalteten Katalysatoreinheiten ausgebildet sein.

Die Abgasreinigungseinrichtung 1 weist ferner eine Brennstoff-Zugabeeinrichtung 5 stromauf des Oxidationskatalysators 2 auf. Über die Brennstoff-Zugabeeinrichtung 5 kann ein kohlenwasserstoff- und/oder wasserstoffhaltiger Brennstoff in den Abgasstrang 9 eingebracht werden. Vorzugsweise ist die Brennstoff-Zugabeeinrichtung 5 dazu vorgesehen, einen an Bord des Fahrzeugs vorhandenen, insbesondere zu dessen Antrieb vorgesehenen Kraftstoff dem Abgas zuzuführen.

Über eine Reduktionsmittel-Zugabeeinrichtung 6 kann dem Abgas ein NH₃ in freier oder gebundener Form aufweisendes Reduktionsmittel zur selektiven katalytischen NOx-Reduktion zugeführt werden. Ohne Einschränkung der Allgemeinheit wird nachfolgend davon ausgegangen, dass es sich bei dem Reduktionsmittel um eine wässrige Harnstofflösung (HWL) handelt. Die HWL weist bevorzugt eine Harnstoff-Sollkonzentration von etwa 32 % auf und wird einem nicht dargestellten Vorratsbehälter entnommen.

Weiterhin ist ein erster in Bezug auf NOx empfindlicher Sensor 7 stromauf des SCR-Katalysators 4 vorgesehen. Wie dargestellt, ist dieser zwischen der Brennstoff-Zugabeeinrichtung 5 und dem Oxidationskatalysator 2 in der Abgasleitung 9 angeordnet. Eine Anordnung an einer anderen Stelle stromauf des SCR-Katalysators 4, beispielsweise zwischen dem Partikelfilter 3 und der Reduktionsmittel-Zugabeeinrichtung 6 kann ebenfalls vorgesehen sein. Ein weiterer, zweiter NOx-Sensor 8 ist stromab des SCR-Katalysators 4 in der Abgasleitung 9 angeordnet. Die NOx-Sensoren sind dabei bevorzugt von gleicher Bauart. Vorzugsweise wird durch Auswertung der von den NOx-Sensoren 7, 8 abgegebenen Signale der NOx-Umsatz des SCR-Katalysators 4 ermittelt und eine HWL-Zugabemenge zur Stickoxidreduktion am SCR-Katalysator entsprechend eingestellt. Hierfür ist vorzugsweise ein nicht dargestelltes Steuergerät vorgesehen, welches die Signale der NOx-Sensoren 7, 8 empfängt und verarbeiten kann. Diese Steuergerät kann bevorzugt außerdem den Betrieb der Zugabeeinrichtungen 5, 6 steuern und weitere Steuerungs- und Auswertefunktionen betreffend den Betrieb der Brennkraftmaschine und der Abgasreinigungsanlage 1 ausführen.

Es versteht sich, dass die Abgasreinigungsanlage 1 weitere hier nicht gesondert dargestellte der Abgasreinigung dienende katalytische oder filterwirksame Komponenten aufweisen kann. Insbesondere kann ein Stickoxid-Speicherkatalysator stromauf des Oxidationskatalysators und/oder zwischen dem Oxidationskatalysator 2 und dem Partikelfilter 3 und/oder zwischen dem Partikelfilter 3 und dem SCR-Katalysator 4 vorgesehen sein. Besonders bevorzugt ist ein Ammoniak-Sperrkatalysator zur Oxidation von NH₃-Schlupf hinter dem SCR-Katalysator 4 vorgesehen. Weiterhin sind vorzugsweise zusätzliche, nicht dargestellte Abgas-, Druck- und Temperatursensoren vorgesehen, über deren Signale der Betrieb der Abgasreinigungsanlage 1 und der Brennkraftmaschine bedarfsgerecht eingestellt werden kann. Temperatursensoren sind bevorzugt jeweils vor und hinter dem Oxidationskatalysator 2 und/oder dem Partikelfilter 3 und/oder dem SCR-Katalysator 4 vorgesehen.

Der Oxidationskatalysator 2 ist bevorzugt als so genannter Diesel-Oxidationskatalysator mit einer Beschichtung mit geringer oder fehlender Sauerstoffspeicherfähigkeit ausgebildet. Es kann auch eine Beschichtung mit einer Drei-Wege-Katalysator-Funktion vorgesehen sein. Ein Träger für die Beschichtung ist vorzugsweise als Keramikträger oder als Metallfolien-Trägerkörper ausgebildet. Es kann zudem ein unmittelbar vorgeschaltetes Heizelement, bevorzugt als beschichteter Metallfolien-Trägerkörper (so genannter E-Kat) ausgebildet, vorgesehen sein.

Der Partikelfilter 3 kann in Sintermetallausführung oder als wanddurchströmte Filtereinheit in Wabenkörperbauweise ausgebildet sein. Vorzugsweise ist für den Partikelfilter 3 eine katalytische Beschichtung, beispielsweise mit einem oxidationskatalytisch wirksamen Material und/oder mit einem SCR-Katalysatormaterial vorgesehen. Das SCR-Katalysatormaterial kann unter oxidierenden Bedingungen eine selektive, kontinuierliche Reduktion von NOx mittels eingespeichertem und/oder zugeführtem Ammoniak (NH₃) als selektivem NOx-Reduktionsmittel katalysieren. Bevorzugt als Katalysatormaterial ist ein Eisen oder Kupfer enthaltender Zeolith. Das SCR-Katalysatormaterial kann dabei auf der Rohgasseite und/oder auf der Reingasseite der filterwirksamen Flächen des Partikelfilters 3 vorgesehen sein. Bei der bevorzugten Ausführung des Partikelfilters 3 als üblicher wanddurchströmter Filter in Wabenkörperbauweise ist eine gegebenenfalls vorhandene Beschichtung mit dem entsprechenden SCR-Katalysatormaterial bevorzugt auf den rohgasexponierten Kanalwänden vorgesehen. Insbesondere in diesem Fall kann es vorteilhaft sein, die SCR-Katalysatorbeschichtung lediglich abschnittsweise eintrittsseitig oder austrittsseitig auf Kanalwänden des Partikelfilters 3 vorzusehen. Beispielsweise kann über einen ersten Teil der axialen Ausdehnung des Partikelfilters 3 von etwa 50 % der Länge eine Beschichtung mit dem SCR-Katalysatormaterial vorgesehen sein. Der in axialer Richtung gesehen hintere Teil kann unbeschichtet oder mit einer oxidationskatalytisch wirksamen Beschichtung versehen sein.

Der stromab des Partikelfilters 3 angeordnete SCR-Katalysator 4 ist analog zu einer gegebenenfalls vorhandenen SCR-Beschichtung des Partikelfilters 3 zur selektiven Reduktion von NOx mit NH₃ befähigt und bezüglich einer solchen Beschichtung bevorzugt gleichartig ausgebildet. Es können auch zwei in Abgasströmungsrichtung hintereinander angeordnete, unterschiedliche SCR-Beschichtungen mit unterschiedlichem Temperaturbereich für maximale Wirksamkeit auf ein und demselben Trägerkörper oder auf zwei mit kurzem Abstand hintereinander angeordneten Trägerkörpern vorgesehen sein. Bevorzugt sind gezonte Beschichtungen umfassend einen Eisen und einen Kupfer enthaltenden Zeolith. Vorzugsweise ist der SCR-Katalysator 4 motorfern im Unterbodenbereich des Fahrzeugs angeordnet. Besonders bevorzugt ist jedenfalls eine Anordnung im Abgasstrang 9 derart, dass bei einer thermischen Partikelfilterregeneration die Temperatur des SCR-Katalysators 4 nicht über 650 °C ansteigt. Durch geometrische Entfernung vom Partikelfilter 3 in Verbindung mit einer entsprechenden Dimensionierung der Abgasleitung 9 in Bezug auf Wärmeabgabe kann erreicht werden, dass zwischen einem auf etwa 800 °C aufgeheizten Partikelfilter 3 und dem SCR-Katalysator 4 ein Temperaturgefälle von mehr als 200 °C oder mehr besteht.

Zur Durchführung einer thermischen Regeneration des Partikelfilters 3 durch Rußabbrand wird die Brennstoff-Zugabeeinrichtung 5 aktiviert und Brennstoff, insbesondere Dieselkraftstoff dem Abgas zugegeben. Durch Oxidation des Brennstoffs am Oxidationskatalysator 2 wird das dem Partikelfilter 3 zugeführte Abgas und der Partikelfilter 3 auf eine für einen Rußabbrand erforderliche Temperatur von etwa 650 °C bis 850 °C aufgeheizt. Meist ist für die gesamte Dauer der thermischen Partikelfilterregeneration eine Brennstoffzufuhr, gegebenenfalls mit Unterbrechungen erforderlich, um die Temperatur des Partikelfilters 3 auf der für einen Rußabbrand erforderlichen Temperatur zu halten.

Die Signale der beiden NOx-Sensoren 7, 8 werden in Bezug auf verschiedene Funktionen, wie beispielsweise zur Ermittlung einer HWL-Dosiermenge, einer Alterungsbestimmung von Oxidationskatalysator 2, Partikelfilter 3 und/oder SCR-Katalysator 4 und verschiedener anderer Diagnosefunktionen ausgewertet. Aus diesem Grund kommt der Zuverlässigkeit und der Genauigkeit der bereitgestellten Signale bzw. Messwert eine besondere Bedeutung zu. Die Genauigkeit der Signale in Bezug auf einen die NOx-Konzentration im Abgas anzeigenden Messwert ist jedoch typischerweise durch Toleranzen von bis etwa +/- 15 % eingeschränkt. Daneben existieren meist mehr oder weniger starke Querempfindlichkeiten, insbesondere gegenüber HC und NH₃. Zudem sind auch die Empfindlichkeiten gegenüber NO und NO₂ typischerweise unterschiedlich. Nachfolgend wird erläutert, wie unter besonderer Berücksichtigung der Querempfindlichkeiten ein Sensorabgleich insbesondere in Bezug auf die Anzeigeempfindlichkeit gegenüber NOx bevorzugt vorgenommen wird.

Der Sensorabgleich erfolgt erfindungsgemäß unmittelbar oder mit kurzem zeitlichem Abstand nach einer thermischen Regeneration des Partikelfilters 3. Bei dieser wird die Temperatur des Partikelfilters 3 auf einen für einen Rußabbrand mit Sauerstoff ausreichenden Wert angehoben. Typischerweise wird der Partikelfilter auf 550 °C oder mehr aufgeheizt. Hierzu werden zunächst innermotorische Maßnahmen, wie beispielsweise eine frühe Kraftstoffnacheinspritzung, ergriffen. Diese Maßnahmen bleiben bevorzugt aufrechterhalten, bis der Oxidationskatalysator 2 Kohlenwasserstoffe zuverlässig oxidativ umsetzen kann. Daraufhin wird die Brennstoff-Zugabeeinrichtung 5 aktiviert und Kraftstoff dem Abgas zugeführt. Dies führt durch Oxidieren des zugegebenen Kraftstoffs am Oxidationskatalysator 2 zu einer weiteren Aufheizung des Partikelfilters 3 auf eine für einen Rußabbrand erforderlich Temperatur.

Die Bedingungen für die thermische Partikelfilter-Regeneration bleiben vorzugsweise solange aufrechterhalten, bis im Partikelfilter 3 angesammelter Ruß weitestgehend oder bis zu einem vorgebbaren Grad entfernt ist. In dieser Zeit ist ein Statusbit des Steuergeräts gesetzt, wodurch eine aktive Partikelfilter-Regeneration angezeigt wird. Die Partikelfilter-Regeneration kann bis zu 15 min oder auch eine längere Zeitspanne dauern. Mit Beendigung der Partikelfilter-Regeneration, d.h. nach Zurücksetzen des entsprechenden Statusbits, liegen die für einen Vergleich der Messwerte und für eine gegebenenfalls erforderliche Messwert- oder Signalkorrektur bzw. einen Empfindlichkeitsabgleich der NOx-Sensoren 7, 8 bevorzugten Bedingungen vor. Die Bedingungen sind derart, dass davon ausgegangen werden kann, dass der erste NOx-Sensor 7 und der zweite NOx-Sensor 8 gleiche Messwerte liefern. Zu den Bedingungen gehört im Einzelnen:

Die NOx-Konzentrationen im Abgas sind am Einbauort der NOx-Sensoren 7, 8 wenigstens annähernd gleich und entsprechen weitestgehend der NOx-Rohemission der Brennkraftmaschine. Dies ist vorliegend deshalb gewährleistet, weil der Abgasstrang weitestgehend frei von Bestandteilen ist, welche NOx reduzieren können. Genauer gesagt ist eine Zufuhr von HC, CO und/oder H₂ enthaltendem Brennstoff über die Brennstoff-Zugabeeinrichtung 5 beendet und die Brennkraftmaschine wird mager und mit wenigstens annähernd vollständiger Kraftstoffverbrennung betrieben. Damit ist jedoch auch die weitere Bedingung erfüllt, dass die NOx-Sensoren 7, 8 mit Abgas beaufschlagt werden, welches weitgehend frei von reduzierenden Bestandteilen ist, welche aufgrund von Querempfindlichkeiten die Messwerte der NOx-Sensoren 7, 8 beeinflussen oder mit NOx reduktiv reagieren könnten. Insbesondere liegt die HC-Konzentration im Abgas unterhalb eines vorgebbaren Grenzwerts von 10 ppm oder niedriger.

Eine HWL-Dosierung ist spätestens zu diesem Zeitpunkt beendet. Vorzugsweise wurde sie bereits vorher, beispielsweise mit Abschluss der innermotorischen Heizmaßnahmen zur Aufheizung des Partikelfilters 3 beendet. Aufgrund der vorangegangenen Partikelfilter-Regeneration weist der SCR-Katalysator 4 eine erhöhte Temperatur von wenigstens 400 °C auf, die typischerweise ebenfalls wenigstens einige Minuten vorhanden war. Damit ist gewährleistet, dass gegebenenfalls im SCR-Katalysator 4 adsorbiertes NH₃ wenigstens annähernd vollständig desorbiert und der SCR-Katalysator 4 somit weitestgehend frei von gespeichertem NH₃ ist. Aufgrund der vergleichsweise hohen Temperaturen der Partikelfilter-Regeneration sind gegebenenfalls im Abgasstrang 9 vorhandene Harnstoffablagerungen verdampft. Somit ist gewährleistet, dass die NH₃-Konzentration im Abgas im gesamten Abgasstrang 9 und insbesondere auch am Ort des zweiten NOx-Sensors 8 vernachlässigbar ist, bzw. unter einem Grenzwert von vorzugsweise 10 ppm oder niedriger liegt. Damit sind insgesamt eine Beeinflussung des Signals des zweiten NOx-Sensors 8 durch NH₃ und ein durch den SCR-Katalysator 4 ermöglichter NOx-Umsatz vermieden.

Ferner ist eine Grenztemperatur von wenigstens 400 °C, vorzugsweise wenigstens 450 °C und besonders bevorzugt von wenigstens 500 °C am Ort des ersten NOx-Sensors 7 und/oder am Ort des zweiten NOx-Sensors 8 überschritten. Damit ist der NO₂-Anteil des im Abgas enthaltenen NOx aus thermodynamischen Gründen vergleichsweise niedrig und eine gegebenenfalls vorhandene unterschiedliche Empfindlichkeit der NOx-Sensoren 7, 8 gegenüber NO und NO₂ schlägt auf das jeweilige Mess-Signal nicht oder nur in geringem Maß durch.

Insgesamt ist somit zu erwarten, dass die NOx-Sensoren 7, 8 Signale derselben Größe abgegeben, bzw. dieselben Messwerte vorliegen. Unter diesen Bedingungen, vorzugsweise unmittelbar oder mit einem vorgebbaren kurzem Zeitabstand nach Beendigung einer thermischen Partikelfilter-Regeneration werden daher Messwerte der NOx-Sensoren 7, 8 wenigstens annähernd zeitgleich erfasst und anschließend miteinander verglichen. Dabei ist es bevorzugt, ein Messwertkollektiv von mehreren kurz hintereinander erfassten Messwerten zu mitteln.

Liegen die Messwerte mehr als ein vorgebbares Maß von beispielsweise 15 % auseinander, so kann davon ausgegangen werden, dass zumindest einer der beiden NOx-Sensoren 7, 8 fehlerhaft ist. In diesem Fall kann die Ausgabe eines Fehlersignals vorgesehen sein. Liegen die Messwerte weniger stark auseinander, so wird ein Messwertabgleich vorgenommen. Dabei kann der Messwert eines der NOx-Sensoren 7, 8, derart korrigiert werden, dass er dem unverändert gelassenen Messwert des anderen NOx-Sensors entspricht. Es kann auch vorgesehen sein, den Messwert des NOx-Sensors, welcher näher an einem rechnerisch für den vorhandenen Motorbetriebspunkt ermittelten NOx-Rohemissionswert liegt, als Bezugswert vorzugeben, und den Messwert des anderen NOx-Sensors auf diesen Wert zu korrigieren. Es können auch beide Messwerte verändert, vorzugsweise auf den gemeinsamen Mittelwert korrigiert werden. In jedem Fall sind die Empfindlichkeiten der NOx-Sensoren 7, 8 gegenüber NOx also nach Durchführen des Abgleichs bzw. der Korrektur gleich groß. Die Korrektur kann am Sensor selbst oder im angeschlossenen Steuergerät, welches die Signale verarbeitet und aufbereitet, vorgenommen werden.

Durch das erläuterte erfindungsgemäße Verfahren sind ein Toleranzausgleich der NOx-Sensoren 7, 8 sowie in der Folge eine verbesserte HWL-Dosiergenauigkeit sowie eine zuverlässigere Überwachung nahezu der gesamten Abgasreinigungsanlage 1 ermöglicht.

## Patentansprüche

1. Verfahren zur Verarbeitung von Messwerten eines ersten Stickoxid-Sensors (7), angeordnet stromauf eines Stickoxidreduktionskatalysators (4) im Abgasstrang (9) eines Kraftfahrzeugs, und von Messwerten eines zweiten Stickoxid-Sensors (8), angeordnet stromab des Stickoxidreduktionskatalysators (4) im Abgasstrang (9) des Kraftfahrzeugs, bei welchem wenigstens annähernd zeitgleich Messwerte des ersten und des zweiten Stickoxid-Sensors (7; 8) erfasst und miteinander verglichen werden,
**dadurch gekennzeichnet, dass**
der Vergleich zu einem Zeitpunkt vorgenommen wird, bei dem am Einbauort des ersten Stickoxid-Sensors (7) und am Einbauort des zweiten Stickoxid-Sensors (8) wenigstens annähernd gleiche Konzentrationen von NOx im Abgas vorhanden sind, wobei eine Empfindlichkeit des ersten Stickoxid-Sensors (7) und/oder des zweiten Stickoxid-Sensors (8) in Abhängigkeit vom Ergebnis des Vergleichs verändert wird, wobei die Empfindlichkeit des ersten Stickoxid-Sensors (7) und/oder des zweiten Stickoxid-Sensors (8) derart verändert wird, dass der erste Stickoxid-Sensor (7) und der zweite Stickoxid-Sensor (8) wenigstens annähernd gleiche Empfindlichkeiten gegenüber NO und/oder N02 aufweisen.

2. Verfahren nach Anspruch 1,
**dadurch gekennzeichnet, dass**
die Empfindlichkeit des ersten Stickoxid-Sensors (7) oder des zweiten Stickoxid-Sensors (8) derart verändert werden, dass der erste Stickoxid-Sensor (7) oder der zweite Stickoxid-Sensor (8) in seiner Empfindlichkeit an die unverändert gelassene Empfindlichkeit des jeweils anderen Stickoxid-Sensors (7, 8) angepasst wird.

3. Verfahren nach Anspruch 1,
**dadurch gekennzeichnet, dass**
die Empfindlichkeit des ersten Stickoxid-Sensors (7) oder des zweiten Stickoxid-Sensors (8) derart verändert werden, dass die Empfindlichkeit des Stickoxid-Sensors (7, 8), dessen Messwert stärker von einem rechnerischen NOx-Rohemissionswert abweicht, an die Empfindlichkeit des anderen Stickoxid-Sensors (2, 8) angepasst wird.

4. Verfahren nach Anspruch 1,
**dadurch gekennzeichnet, dass**
die Empfindlichkeit des ersten Stickoxid-Sensors (7) und des zweiten Stickoxid-Sensors (8) derart verändert werden, dass die Messwerte der Stickoxid-Sensoren (7, 8) einem Mittelwert der Messwerte der Stickoxid-Sensoren (7, 8) vor Durchführung der Empfindlichkeitsveränderung entsprechen.

5. Verfahren nach einem der Ansprüche 1 bis 4, **dadurch gekennzeichnet, dass** der Vergleich zu einem Zeitpunkt vorgenommen wird, bei dem am Einbauort des ersten Stickoxid-Sensors (7) und am Einbauort des zweiten Stickoxid-Sensors (8) die Konzentrationen von Kohlenwasserstoffen und/oder Ammoniak im Abgas vorgegebene Grenzwerte von jeweils weniger als 20 ppm nicht überschreiten.

6. Verfahren nach einem der Ansprüche 1 bis 5, **dadurch gekennzeichnet, dass** der Vergleich zu einem Zeitpunkt vorgenommen wird, bei dem die Abgastemperatur am Einbauort des ersten Stickoxid-Sensors (7) und am Einbauort des zweiten Stickoxid-Sensors (8) 450 °C überschreitet.

7. Verfahren nach einem der Ansprüche 1 bis 6, **dadurch gekennzeichnet, dass** ein Fehlersignal ausgegeben wird, wenn die Messwerte der Stickoxid-Sensoren (7, 8) um mehr als ein vorgebbares Maß voneinander abweichen..

8. Verfahren nach einem der Ansprüche 1 bis 6, **dadurch gekennzeichnet, dass**
der Vergleich unmittelbar im Anschluss an eine thermische Regeneration eines im Abgasstrang (9) angeordneten Partikelfilters (3) erfolgt.

9. Verfahren nach einem der Ansprüche 1 bis 8, **dadurch gekennzeichnet, dass**
im Abgasstrang (8) in Abgasströmungsrichtung hintereinander ein Oxidationskatalysator (2), ein Partikelfilter (3) und der Stickoxidreduktionskatalysators (4) angeordnet sind, und der erste Stickoxid-Sensor (7) eingangsseitig des Oxidationskatalysators (2) und der zweite Stickoxid-Sensor (8) stromab des Stickoxidreduktionskatalysators (4) angeordnet ist.

## Claims

1. Method for processing measured values from a first nitrogen oxide sensor (7) located upstream of a nitrogen oxide reducing catalyst (4) in the exhaust system (9) of a motor vehicle and of measured values from a second nitrogen oxide sensor (8) located downstream of the nitrogen oxide reducing catalyst (4) in the exhaust system (9) of the motor vehicle, wherein measured values of the first and the second nitrogen oxide sensor (7; 8) are detected at least approximately simultaneously and compared to each other,
**characterised in that**
the comparison is carried out at a point in time when an at least approximately equal NOₓ concentration is present in the exhaust gas at the installation location of the first nitrogen oxide sensor (7) and at the installation location of the second nitrogen oxide sensor (8), wherein a sensitivity of the first nitrogen oxide sensor (7) and/or of the second nitrogen oxide sensor (8) is changed as a function of the result of the comparison, wherein the sensitivity of the first nitrogen oxide sensor (7) and/or of the second nitrogen oxide sensor (8) is changed in such a way that the first nitrogen oxide sensor (7) and the second nitrogen oxide sensor (8) have at least approximately equal sensitivities to NO and N02.

2. Method according to claim 1,
**characterised in that**
the sensitivity of the first nitrogen oxide sensor (7) or of the second nitrogen oxide sensor (8) is changed in such a way that the first nitrogen oxide sensor (7) or the second nitrogen oxide sensor (8) is adapted in its sensitivity to the unchanged sensitivity of the respective other nitrogen oxide sensor (7, 8).

3. Method according to claim 1,
**characterised in that**
the sensitivity of the first nitrogen oxide sensor (7) or of the second nitrogen oxide sensor (8) is changed in such a way that the sensitivity of that nitrogen oxide sensor (7, 8) whose measured value deviates more strongly from a calculated NOₓ raw emission value is adapted to the sensitivity of the other nitrogen oxide sensor (7, 8).

4. Method according to claim 1,
**characterised in that**
the sensitivity of the first nitrogen oxide sensor (7) or of the second nitrogen oxide sensor (8) is changed in such a way that the measured values of the nitrogen oxide sensors (7, 8) correspond to an average value of the measured values of the nitrogen oxide sensors (7, 8) before the sensitivity change.

5. Method according to any of claims 1 to 4,
**characterised in that**
the comparison is carried out at a point in time when the concentrations of hydrocarbons and/or ammonia in the exhaust gas do not exceed preset limit values of less than 20 ppm each at the installation location of the first nitrogen oxide sensor (7) and at the installation location of the second nitrogen oxide sensor (8).

6. Method according to any of claims 1 to 5,
**characterised in that**
the comparison is carried out at a point in time when the exhaust gas temperature exceeds 450°C at the installation location of the first nitrogen oxide sensor (7) and at the installation location of the second nitrogen oxide sensor (8).

7. Method according to any of claims 1 to 6,
**characterised in that**
a fault signal is output if the measured values of the nitrogen oxide sensors (7, 8) deviate from each other by more than a presettable amount.

8. Method according to any of claims 1 to 6,
**characterised in that**
the comparison is carried out immediately after a thermal regeneration of a particulate filter (3) located in the exhaust system (9).

9. Method according to any of claims 1 to 8,
**characterised in that**
an oxidising catalyst (2), a particulate filter (3) and the nitrogen oxide reducing catalyst (4) are arranged one behind the other in the exhaust system (9) in the direction of exhaust gas flow, and **in that** the first nitrogen oxide sensor (7) is located on the inlet side of the oxidising catalyst (2) and the second nitrogen oxide sensor (8) is located downstream of the nitrogen oxide reducing catalyst (4).

## Revendications

1. Procédé de traitement de valeurs de mesure d'un premier capteur des oxydes d'azote (7), disposé en amont d'un catalyseur de réduction des oxydes d'azote (4) dans la ligne de gaz d'échappement (9) d'un véhicule automobile, et de valeurs de mesure d'un second capteur des oxydes d'azote (8), disposé en aval du catalyseur de réduction des oxydes d'azote (4) dans la ligne d'échappement (9) du véhicule automobile, selon lequel au moins des valeurs de mesure approximativement simultanées du premier et du second capteur des oxydes d'azote (7, 8) sont détectées et comparées les unes aux autres, **caractérisé en ce qu'**on réalise la comparaison à un moment où dans le lieu de montage du premier capteur des oxydes d'azote (7) et dans le lieu de montage du second capteur des oxydes d'azote (8) sont présentes des concentrations en NOx au moins approximativement identiques dans le gaz d'échappement, une sensibilité du premier capteur des oxydes d'azote (7) et/ou du second capteur des oxydes d'azote (8) étant modifiée en fonction du résultat de la comparaison, la sensibilité du premier capteur des oxydes d'azote (7) et/ou du second capteur des oxydes d'azote (8) étant modifiée de manière que le premier capteur des oxydes d'azote (7) et le second capteur des oxydes d'azote (8) présentent des sensibilités au moins approximativement identiques par rapport au NO et/ou N02.

2. Procédé selon la revendication 1, **caractérisé en ce que** la sensibilité du premier capteur des oxydes d'azote (7) ou du second capteur des oxydes d'azote (8) est modifiée de manière que le premier capteur des oxydes d'azote (7) ou le second capteur des oxydes d'azote (8) quant à sa sensibilité est adapté à la sensibilité inchangée de l'autre capteur des oxydes d'azote respectif (7, 8).

3. Procédé selon la revendication 1, **caractérisé en ce que** la sensibilité du premier capteur des oxydes d'azote (7) ou du second capteur des oxydes d'azote (8) est modifiée de manière que la sensibilité du capteur des oxydes d'azote (7, 8), dont la valeur de mesure est très différente d'une valeur d'émission brute de NOx calculée, soit adaptée à la sensibilité de l'autre capteur des oxydes d'azote (2, 8).

4. Procédé selon la revendication 1, **caractérisé en ce que** la sensibilité du premier capteur des oxydes d'azote (7) et du second capteur des oxydes d'azote (8) est modifiée de manière que les valeurs de mesure des capteurs des oxydes d'azote (7, 8) correspondent à une valeur moyenne des valeurs de mesure des capteurs des oxydes d'azote (7, 8) avant que la sensibilité ne soit modifiée.

5. Procédé selon l'une quelconque des revendications 1 à 4, **caractérisé en ce qu'**on réalise la comparaison à un moment, où dans le lieu de montage du premier capteur des oxydes d'azote (7) et dans le lieu de montage du second capteur des oxydes d'azote (8) les concentrations en hydrocarbures et/ou en ammoniac dans le gaz d'échappement ne passent pas au-dessus des valeurs limites prédéfinies respectivement inférieures à 20 ppm.

6. Procédé selon l'une quelconque des revendications 1 à 5, **caractérisé en ce qu'**on réalise la comparaison à un moment où la température de gaz d'échappement dans le lieu de montage du premier capteur des oxydes d'azote (7) et dans le lieu de montage du second capteur des oxydes d'azote (8) passe au-dessus de 450°C.

7. Procédé selon l'une quelconque des revendications 1 à 6, **caractérisé en ce qu'**un signal d'erreur est émis lorsque les valeurs de mesure des capteurs des oxydes d'azote (7, 8) sont différentes entre elles dans une mesure prédéfinie.

8. Procédé selon l'une quelconque des revendications 1 à 6, **caractérisé en ce que** la comparaison s'effectue immédiatement à la suite d'une régénération thermique d'un filtre à particules (3) disposé dans la ligne d'échappement (9).

9. Procédé selon l'une quelconque des revendications 1 à 8, **caractérisé en ce que** dans la ligne d'échappement (8) sont disposés dans la direction d'écoulement de gaz d'échappement les uns derrière les autres un catalyseur d'oxydation (2), un filtre à particules (3) et le catalyseur de réduction des oxydes d'azote (4) et le premier capteur des oxydes d'azote (7) est disposé côté entrée du catalyseur d'oxydation (2) et le second capteur des oxydes d'azote (8) est disposé en aval du catalyseur de réduction des oxydes d'azote (4).
